# FASCICULE DE BREVET EUROPEEN

(11) **EP 1 802 571 B1**
(45) Date de publication et mention de la délivrance du brevet: **23.11.2011**
(21) Numéro de dépôt: 05809316.2
(22) Date de dépôt: 10.10.2005
(51) Int. Cl.: C07D 205/04, A61K 31/397, A61P 3/00

(54) **PROCEDE ET INTERMEDIAIRES DE PREPARATION DE DERIVES DE N-(1-BENZHYDRYL-AZETIDIN-3-YL)-N-PHENYL-METHYLSULFONAMIDE**
VERFAHREN UND ZWISCHENPRODUKTE FÜR DIE HERSTELLUNG VON N-(1-BENZHYDRYL-AZETIDIN-3-YL)-N-PHENYL-METHYLSULPHONAMID-DERIVATEN
METHOD AND INTERMEDIATES FOR THE PREPARATION OF DERIVATIVES OF N-(1-BENZHYDRYL-AZETIDIN-3-YL)-N-PHENYL-METHYLSULPHONAMIDE

(30) Priorité: 14.10.2004 FR 0410845
(43) Date de publication de la demande: 04.07.2007
(73) Titulaire: Aventis Pharma S.A., 92160 Antony (FR)
(72) Inventeur: BOFFELLI, Philippe, F-93110 ROSNY SOUS BOIS (FR); DELTHIL, Michel, F-93130 NOISY LE SEC (FR); GRONDARD, Luc, F-91080 COURCOURONNES (FR); LAMPILAS, Maxime, F-93230 ROMAINVILLE (FR); MALPART, Joël, F-45160 OLIVET (FR); MUTTI, Stéphane, F-94170 LE PERREUX SUR MARNE (FR); NAIT BOUDA, Lahlou, F-93140 BONDY (FR); RIEKE-ZAPP, Joerg, 60325 FRANKFURT AM MAIN (DE)
(74) Mandataire: Morel-Pécheux, Muriel
(86) Numéro de dépôt international: PCT/FR2005/002489
(87) Numéro de publication internationale: WO 2006/040464

(56) Documents cités:
- WO-A-01/64634
- WO-A-02/28346
- ZUBOVICS, ZOLTAN ET AL: "Synthesis and antiarrhythmic activity of N-arylalkylenediamines" EUROPEAN JOURNAL OF MEDICINAL CHEMISTRY , 21(5), 370-8 CODEN: EJMCA5; ISSN: 0223-5234, 1986, XP002332900
- GAKHAR, H. K. ET AL: "A new method of preparation of 1,3-dioxolo[4,5-h][1,4]benzodiazepin- 9-ones" INDIAN JOURNAL OF CHEMISTRY, SECTION B: ORGANIC CHEMISTRY INCLUDING MEDICINAL CHEMISTRY , 34B(1), 48-50 CODEN: IJSBDB; ISSN: 0376-4699, 1995, XP008048918

## Description

La présente invention concerne les procédés de préparation de dérivés d'azétidine de type N-(1-benzhydryl-azetidin-3-yl)-N-phényl-méthylsulfonamide (I) et en particulier le N-{1-[bis-(4-chlorophényl)méthyl]azétidin-3-yl} N-(3,5-difluorophényl)-méthylsulfonamide. Ces produits sont décrits dans la demande de brevet WO 0164634. Ces produits sont connus pour présenter une forte affinité pour les récepteurs cannabinoïdes et particulièrement ceux de type CB1 et sont donc utiles dans le traitement et la prévention des désordres touchant au système nerveux central, au système immunitaire, au système cardio-vasculaire ou endocrinien, au système respiratoire et aux désordres de la reproduction. C'est ainsi que ces composés peuvent être utilisés pour le traitement ou la prévention des psychoses y compris la schizophrénie, des troubles anxieux, de la dépression, de l'épilepsie, de la neurodégénération ou neurodégénérescence, des désordres cérébelleux et spinocérébelleux, des désordres cognitifs, du traumatisme crânien, des attaques de panique, des neuropathies périphériques, des glaucomes, de la migraine, de la maladie de Parkinson, de la maladie d'Alzheimer, de la chorée de Huntington, du syndrome de Raynaud, des tremblements, du désordre compulso-obsessionnel, de la démence sénile, des désordres thymiques, du syndrome de Tourette, de la dyskinésie tardive, des désordres bipolaires, des cancers, des désordres du mouvement induit par les médicaments, des dystonies, des chocs endotoxémiques, des chocs hémorragiques, de l'hypotension, de l'insomnie, des maladies immunologiques, de la sclérose en plaques, des vomissements, de l'asthme, des troubles de l'appétit (boulimie, anorexie), de l'obésité, des troubles de la mémoire, dans le sevrage aux traitements chroniques et abus d'alcool ou de médicaments (opioides, barbituriques, cannabis, cocaïne, amphétamine, phencyclide, hallucinogènes, benzodiazépines par exemple), comme analgésiques ou potentialisateurs de l'activité analgésique des médicaments narcotiques et non narcotiques.

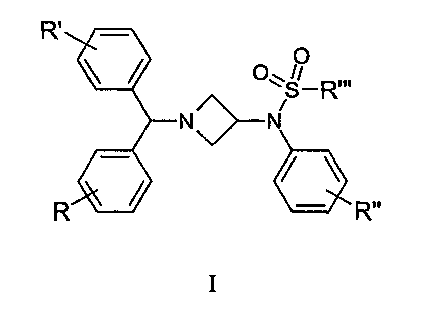

La présente invention concerne la préparation de dérivés de formule générale (I) N-(1-benzhydryl-azetidin-3-yl)-N-phényl-méthylsulfonamide dans laquelle R et R' R" représentent indépendamment l'un de l'autre un ou plusieurs radicaux hydrogène, halogène (Cl, F, Br, I), cyano, nitro, alkyle linéaire ou ramifié de 1 à 6 atomes de carbone; alkoxy linéaire ou ramifié de 1 à 6 atomes de carbone, carboxylate d'alkyle linéaire ou ramifié de 1 à 6 atomes de carbone, trifluorométhyle, trifluorométhoxy et R"' représente un groupe alkyle ou perfluoroalkyle linéaire ou ramifié de 1 à 6 atome de carbone ou un groupement aryle éventuellement substitué par un ou plusieurs radicaux R".

La demande de brevet WO 0164634 décrit des voies d'accès général pour l'obtention de tels produits et en particulier une synthèse par condensation d'un sulfonate et d'un sulfonamide au sein d'un solvant inerte tel que le dioxanne en présence de Cs₂CO₃ au reflux du mélange réactionnel.

Le composé de formule (I) est obtenu par condensation d'un sulfonate de formule générale (II) et un sulfonamide de formule générale (III) dans lesquelles R et R' R" représentent indépendamment l'un de l'autre un ou plusieurs radicaux hydrogène, halogène (Cl, F, Br, I), cyano, nitro, alkyle linéaire ou ramifié de 1 à 6 atomes de carbone, alkoxy linéaire ou ramifié de 1 à 6 atomes de carbone, carboxylate d'alkyle linéaire ou ramifié de 1 à 6 atomes de carbone, trifluorométhyle, trifluorométhoxy, R"' représente un groupe alkyle ou perfluoroalkyle linéaire ou ramifié de 1 à 6 atome de carbone, ou un groupement aryle éventuellement substitué par un ou plusieurs radicaux R"
et R1 représente un radical méthyle, trifluorométhyle, C₄F₉, C₈F₁₇ ou phényle éventuellement substitué par un reste méthyle, bromo ou nitro

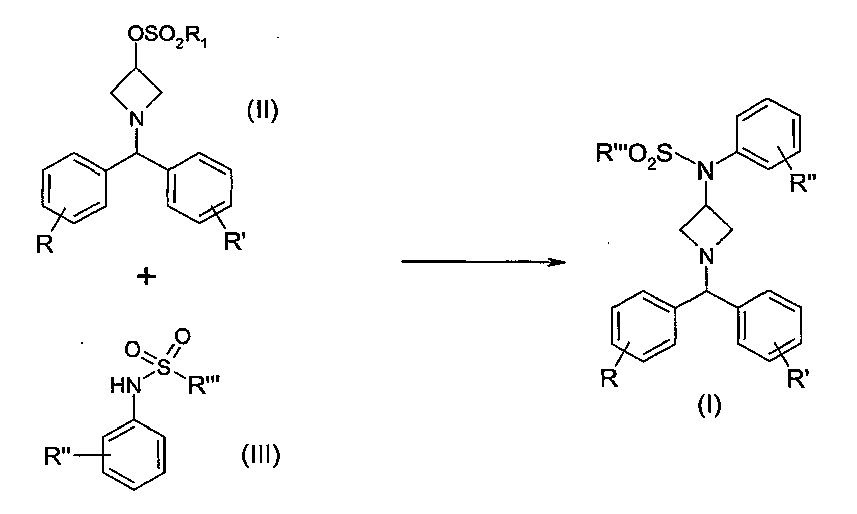

La condensation s'effectue dans un solvant organique éventuellement en présence d'eau, à une température comprise entre 20°C et 150°C en présence d'une base minérale ou organique et d'un agent de transfert de phase.

On opère de préférence au sein d'un solvant tel que le toluène, le xylène. D'autres solvants peuvent être utilisés tels que, l'heptane, l'hexane, un solvant éthéré tel que le tetrahydrofuranne, le diméthoxyéthane, ou encore un solvant chloré tel que le mono ou le dichlorobenzène, le chlorobutane ou le chlorure de méthylène, les alcools tels que le methanol, l'éthanol, l'isopropanol ou le butanol, l'acétonitrile, la pyridine, les solvants aprotiques dipolaires tels que le DMF, le diméthylacétamide ou la N-méthylpyrolidone, les cétones telles que l'acétone, la méthyléthylcétone ou la méthylisobutylcétone. On utilise également une base minérale ou organique telle que l'hydrogénocarbonate, carbonate, phosphate, hydroxyde d'un métal alcalin tel que le lithium, le sodium, le potassium ou le césium et plus particulièrement de phosphate tripotassique anhydre. On peut également utiliser des alkoxydes de métaux alcalins tels que le méthylate, l'éthylate, le t-amylate ou le t-butylate de sodium ou de potassium. On peut enfin utiliser des bases aminées telles que le DBU, DBN ou la tetramétylguanidine. La réaction s'effectue en présence d'un agent de transfert de phase tel qu'un sel d'ammonium quaternaire ou un complexant. Par sel d'ammonium quaternaire on entend un halogénure, un hydroxyde, un sulfate ou hydrogenosulfate de tetraalkylammonium, ou de benzyltrialkylammonium. Les groupes alkyls correspondent à ceux communément utilisés par l'homme du métier tel qu'un groupe alkyle linéaire de 1 à 16 atomes de carbone. Par agent complexant ou de transfert de phase, on entend les éthers couronne tel que le 12C4, 15C5, 18C6, le pentaglyme (Glyme-6) ou le polyéthylène glycol PEG 400 et plus particulièrement le tris(dioxa-3,6-heptyl)amine (TDA-1).

L'invention a également pour objet un procédé, caractérisé en ce que le composé de formule (II) est obtenu en traitant un composé de formule générale (IV) ou un de ces sels d'acide par un chlorure ou un anhydride sulfonique en présence de base à une température comprise entre -60°C et 100°C. Par chlorure ou anhydride sulfonique en entend le chlorure ou l'anhydride méthane sulfonique, les anhydride d'acide perfluoroalcanesulfonique de 1 à 9 atomes de carbone, le chlorure de l'acide benzènesulfonique, p-toluènesulfonique, p-nitrobenzènesulfonique, p-bromobenzènesulfonique. Par sel d'acide on entend un chlorhydrate, bromhydrate, sulfate ou hydrogénosulfate, sulfonate tel que le méthanesulfonate ou le p-toluènesulfonate, ou un sel acide carboxylique tel qu'un acétate, un oxalate ou un fumarate. Préférentiellement R"' est un méthyle.

On opère de préférence au sein d'un solvant tel que le toluène, le xylène, l'heptane, l'hexane, un solvant éthéré tel que le tetrahydrofuranne, le diméthoxyéthane, ou encore un solvant chloré tel que le mono ou le dichlorobenzène, le chlorobutane ou le chlorure de méthylène, l'acétonitrile, les solvants aprotiques dipolaires tels que le DMF, le dimethylacétamide ou la N-methylpyrolidone et en présence d'une base telle que de d'hydrogenocarbonante, de carbonate, de phosphate, d'hydroxyde d'un metal alcalin tel que le lithium, le sodium, le potassium ou le césium. On peut également utiliser des bases aminées telles que la triéthylamine, la diisopropyléthylamine, la N,N-diméthylaniline, le N-méthylimidazole, la pyridine, le DBU, le DBN ou la tétraméthylguanidine.

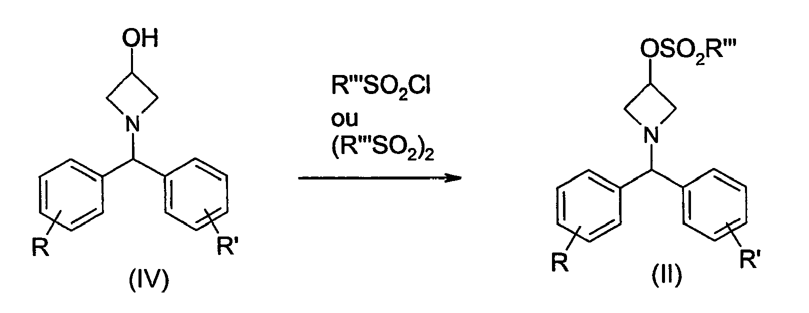

L'invention a également pour objet un procédé, caractérisé en ce que le composé de formule (IV) est obtenu en traitant un composé de formule générale (V) ou un de ses sels d'acide avec de l'épibromhydrine ou épichlorhydrine à une température comprise entre 20°C et 150 °C. 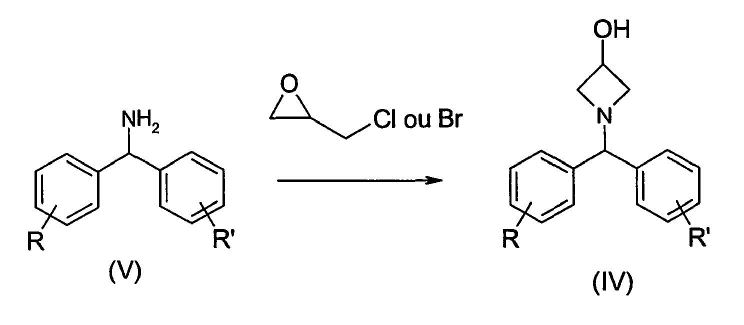 On opère de préférence au sein d'un solvant organique en présence de base, d'un additif et le cas échéant d'une phase aqueuse.

Par sel d'acide on entend un chlorhydrate, bromhydrate, sulfate ou hydrogenosulfate, sulfonate tel que le methanesulfonate ou le p-toluènesulfonate ou un sel d'acide carboxylique tel qu'un acétate, un oxalate ou un fumarate.

On opère de préférence au sein d'un solvant tel que le toluène, le xylène, l'heptane, l'hexane, un solvant éthéré tel que le tetrahydrofuranne, le diméthoxyéthane, ou encore un solvant chloré tel que le mono ou le dichlorobenzène, le chlorobutane ou le chlorure de méthylène, les alcools linéaire ou ramifiés de 1 à 6 atomes de carbone tel que l'éthanol et plus particulièrement le n-butanol. Par base on entend de d'hydrogénocarbonate, du carbonate, du phosphate, de l'hydroxyde d'un métal alcalin tel que le lithium, le sodium, le potassium ou le césium et plus particulièrement de phosphate tripotassique anhydre. On peut également utiliser des alkoydes de métaux alcalins tels que le méthylate, l'éthylate, le t-amylate ou le t-butylate de sodium ou de potassium. On peut enfin utiliser des bases aminées telles que la diisopropyléthylamine, le DBU, DBN ou la tétraméthylguanidine. Par additif, on entend les iodures ou bromures de métaux alcalins ou alcalinoterreux et plus particulièrement l'iodure de sodium.

L'invention a également pour objet un procédé, caractérisé en ce que le composé de formule (V) est obtenu en chauffant un composé de formule générale (VI) en milieu acide.

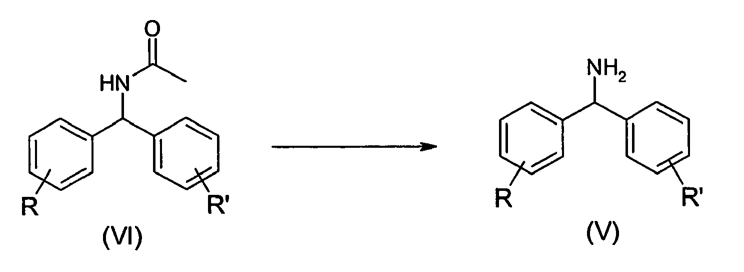

On opère de préférence au sein d'un solvant tel que les alcools linéaires ou ramifiés de 1 à 6 atomes de carbone et plus particulièrement le n-butanol ou en mélange ou non avec de l'eau, en présence d'un acide minéral fort tel que l'acide bromhydrique, sulfurique et plus particulièrement l'acide chlorhydrique à une température comprise entre 80°C et 150°C.

L'invention a également pour objet un procédé, caractérisé en ce que le composé de formule (VI) est obtenu à partir du composé de formule (VII) par une réaction de Ritter en présence d'un nitrile et d'une catalyse acide.

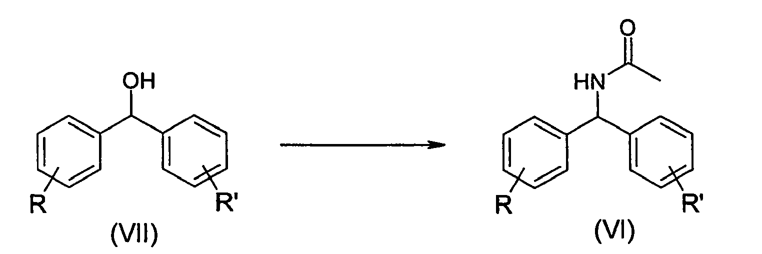

On opère de préférence à l'aide d'un nitrile tel que le propionitrile, le butyronitrile, le benzonitrile, le cyanoacétate d'éthyle et plus particulièrement en présence d'acétonitrile. On réalise un catalyse acide en présence d'acide sulfurique, d'acide méthanesulfonique ou trifluorométhanesulfonique, d'acide acétique ou propionique et plus particulièrement d'acide formique à une température comprise entre 50°C et 150°C.

La présente invention a donc aussi pour objet la synthèse en trois étapes du produit de formule (IV) 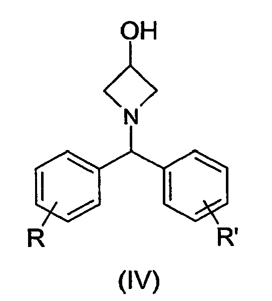 dans laquelle R et R' représentent indépendamment l'un de l'autre un ou plusieurs radicaux hydrogène, halogène (Cl, F, Br, I), cyano, nitro, alkyle linéaire ou ramifié de 1 à 6 atomes de carbone, alkoxy linéaire ou ramifié de 1 à 6 atomes de carbone, carboxylate d'alkyle linéaire ou ramifié de 1 à 6 atomes de carbone, trifluorométhyle, trifluorométhoxy
a) un produit de formule (VII) 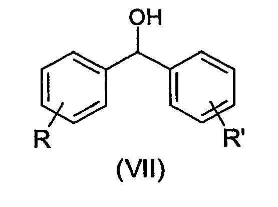 dans laquelle R et R' représentent indépendamment l'un de l'autre un ou plusieurs radicaux hydrogène, halogène (Cl, F, Br, I), cyano, nitro, alkyle linéaire ou ramifié de 1 à 6 atomes de carbone, alkoxy linéaire ou ramifié de 1 à 6 atomes de carbone, carboxylate d'alkyle linéaire ou ramifié de 1 à 6 atomes de carbone, trifluorométhyle, trifluorométhoxy réagit avec de l'acétonitrile en présence d'une catalyse par de l'acide formique à une température comprise entre 50°C et 150°C pour donner le produit de formule (VI) 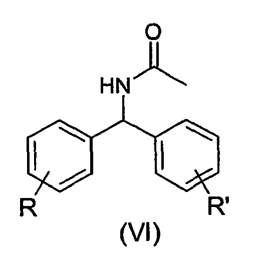 dans laquelle R et R' représentent indépendamment l'un de l'autre un ou plusieurs radicaux hydrogène, halogène (Cl, F, Br, I), cyano, nitro, alkyle linéaire ou ramifié de 1 à 6 atomes de carbone, alkoxy linéaire ou ramifié de 1 à 6 atomes de carbone, carboxylate d'alkyle linéaire ou ramifié de 1 à 6 atomes de carbone, trifluorométhyle, trifluorométhoxy
b) le produit de formule (VI) est transformé en chauffant et en milieu acide en un produit de formule (V) ou un de ces sels d'acide 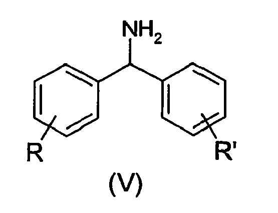 dans laquelle R et R' représentent indépendamment l'un de l'autre un ou plusieurs radicaux hydrogène, halogène (Cl, F, Br, I), cyano, nitro, alkyle linéaire ou ramifié de 1 à 6 atomes de carbone, alkoxy linéaire ou ramifié de 1 à 6 atomes de carbone, carboxylate d'alkyle linéaire ou ramifié de 1 à 6 atomes de carbone, trifluorométhyle, trifluorométhoxy,
c) le produit de formule (V) est transformé en produit de formule (IV) dans un solvant organique le n-butanol en présence de base le phosphate tripotassique anhydre, d'un additif l'iodure de sodium et le cas échéant d'une phase aqueuse.

L'invention a également pour objet un procédé selon ce qui précède, caractérisé en ce que le composé de formule (VII) est obtenu par réduction d'une benzophénone de formule (VIII), en présence d'un agent réducteur à une température de -40°C à +30°C. Suivant la nature de l'agent réducteur, la réaction est réalisée dans les éthers tels que le tetrahydrofurane ou le diméthoxyéthane, les solvants aromatiques tels que le toluène, les solvants chlorés tels que le dichlorométhane, les alcools tels que le méthanol, l'éthanol ou l'eau. Les agents réducteurs utilisés peuvent être le borohydrure de sodium, le borohydrure de lithium), l'hydrure de lithium et d'aluminium, ou l'hydrure de diisobutylaluminium.

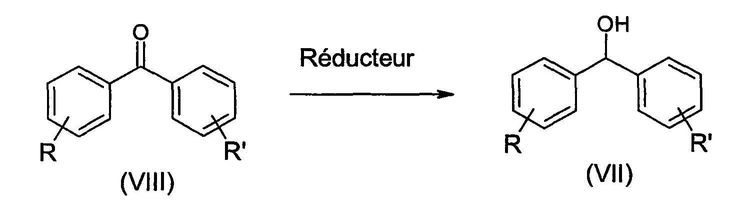

L'intermédiaire (V) peut également être obtenu directement à partir de l'intermédiaire (VIII) en utilisant la réaction de Leuckart via un intermédiaire (IX).

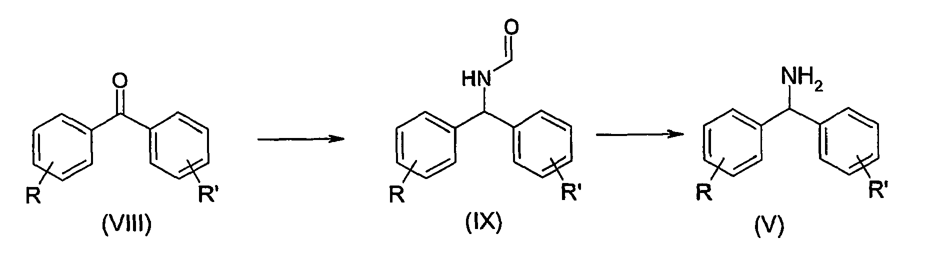

La réaction de formation de (IX) se fait en présence d'acide formique, formiate d'ammonium et de formamide à 170-175°C ou de formamide avec une catalyse au chlorure de magnésium. La deformylation de (IX) est réalisé en présence de HCl dans de l'eau ou du méthanol. La déformylation de (IX) est réalisée dans des conditions analogues à celles utlisées pour la transformation de VI en V.

La présente invention a donc également pour objet un procédé de synthèse du produit (V) caractérisé en ce que
a) formation N-[Bis-(4-chloro-phenyl)-methyl]-formamide (IX) du à partir du Bis-(4-chloro-phenyl)-methanone en présence de formamide avec une catalyse au chlorure de magnésium
b) déformylation en présence de HCl dans du méthanol pour donner le [Bis-(4-chlorophenyl)methyl]-amine.

Par rapport à la demande de brevet WO 0164634, ce nouveau procédé apporte des améliorations en matière de sécurité, ainsi qu'une simplification des étapes de purification et d'isolement des intermédiaires ou du produit fini, notamment par chromatographie sur silice pour les remplacer par des cristallisations, afin de rendre le procédé compatible avec une production industrielle.

A titre d'exemple, le schéma réactionnel ci-dessous illustre de manière non limitative les réactifs utilisés pour chacune des étapes et pour lequel R, R'représentent des chlores en position para, R" représente un fluor en position 3 et 5 R"' est un méthyle, le solvant est le toluène, la base le phosphate tripotassique et l'agent de transfert de phase le tris(dioxa-3,6-heptyl)amine (TDA-1), pour former le N-{1-[bis-(4-chlorophényl)méthyl]azétidin-3-yl}-N-(3,5-difluorophényl)-méthylsulfonamide.

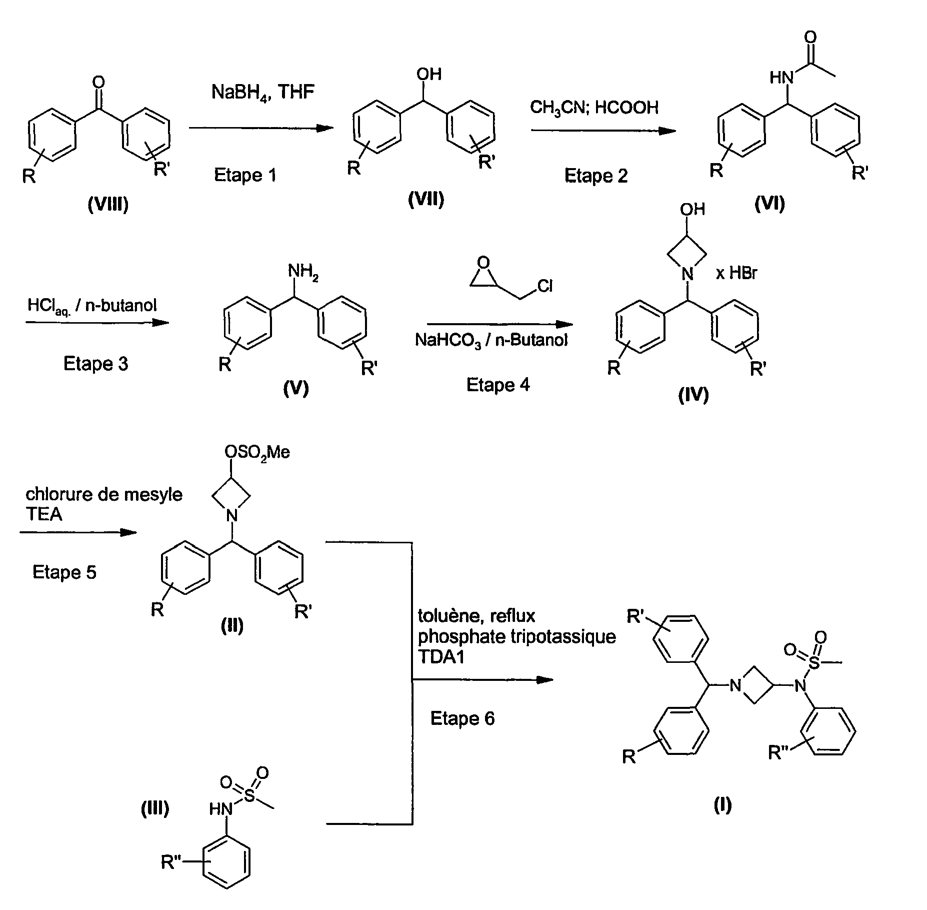

### Partie expérimentale pour la synthèse de N-{1-[bis-(4-chlorophényl méthyl)azétidin-3-yl}-N-(3,5-difluorophényl)-méthylsulfonamide

### Etape 1

Dans un tétracol de 1 litre muni d'une agitation mécanique, d'une sonde thermométrique, placé sous atmosphère d'azote, on introduit à une température de 20+2°C 50g 4, 4' dichlorobenzophenone et 300 mL de THF. 54ml (0,32 mole par mole de produit) de solution d'hydroborure de sodium (solution préparée extemparanément avec 2.7 g de borohydure de sodium, 0.135 ml de lessive de soude à 32 % et 54 ml d'eau déminéralisée) sont ajouté en 1 heure sous azote à 20±2°C.

La solution obtenue est maintenue sous agitation pendant 3h à 20±2°C. Ensuite, 230 ml d'acide chlorhydrique 1N sont ajoutés en 60 minutes à 20°/22°C. La phase aqueuse est décantée et la phase organique contenant le 4,4 Dichlorobenzydrol est utilisée directement dans l'étape 2.

### Etape 2

Dans un tétracol de 2 litres muni d'une agitation mécanique, d'une sonde thermométrique etplacée sous atmosphère, on introduit à 20±2°C la solution de 4,4' Dichlorobenzydrol puis chauffe entre 68° et 75°C et concentré à pression ordinaire à 80 ml. La solution est refroidie à 50°±5°C et 300 mL d'acétonitrile sont ajoutés en une fois.

250 ml d'acétonitrile sont distillés à pression ordinaire puis 100 mL d'acétonitrile sont à nouveau ajoutés ajoutés en une fois. 150 ml d'acétonitrile sont distillé sous agitation, à pression ordinaire.

La solution est ensuite refroidie à 65±5°C et 200 ml d'acide formique sont ajoutés en 10 minutes en maintenant à la température à 65±5°C:

La solution biphasique est maintenue sous bonne agitation, sous courant d'azote azote au reflux (103°C) pendant 5 heures. La solution est refroidie sous agitation à 80°±2°C et 750 mL d'eau déminéralisée sont ajoutés en 30 minutes. La suspension obtenue est maintenu pendant 30 minutes sous agitation, à 80°C°±2°C puis est refroidi à +5°C°/+10°C en 30 minutes et est maintenu à cette température pendant 1 heure.

Le solide est essoré et lavé par empâtage avec 3 x 100 mL d'eau déminéralisée.

On obtient le N[bis(4-chlorophenyl)methyl]acetamide avec un rendement de 95 %.

### Etape 3

Dans un tétracol de 1 litre muni d'une agitation mécanique, d'une sonde thermométrique et placé sous atmosphère d'azote, on introduit 50 g de N[bis(4-chlorophenyl)methyl]acetamide, 250 mL de n-butanol et 150 mL d'eau déminéralisée à 20±2°C.

Puis en 15 min sont ajoutés 135 mL d'acide chlorhydrique à 37 %, le mélange est exothermique et la température monte à 32°C, la dissolution est partielle, et le mélange est biphasique. Le mélange réactionnel est chauffé sous agitation, sous azote au reflux(93°C) pendant 15 h. 125 mL d'eau déminéralisée sont ajoutés en filet en 5 minutes environ sous agitation, sous azote au reflux. 350 ml de milieu réactionnel sont distillés à pression ordinaire. La température évolue jusqu'à 103°C.

Terminer la cristallisation par addition de 125 mL d'eau déminéralisée ajoutée en filet en maintenant au reflux (105°C).

La suspension est refroidie à 10°C'±2°C en 30 minutes puis est maintenu une heure à cette température. Le milieu est essoré et lavé par déplacement avec 50 mL d'eau déminéralisée

On obtient le chlorhydrate de [bis(4-chlorophényl)méthyl]amine avec un rendement de 98 %.

### Etape4

Dans un tétracol de 1 litre muni d'une agitation mécanique, d'une sonde thermométrique et placé sous atmosphère d'azote on introduit 50 g de chlorhydrate de [bis(4-chlorophényl)méthyl]amine, 300 ml de n-butanol et 17.5 g de bicarbonate de sodium à 20±2°C. On obtient une suspension blanche qui est chauffée à 80°±2°C

On maintient à cette température pendant 1 heure. Le milieu est refroidi, à 20°±2°C et 29,6 g d'épichlorhydrine sont ajoutés en filet (environ 5 minutes) à 20±2°C. Le milieu est chauffé sous agitation, sous azote à 80°±2°C pendant 4 heures.

Le milieu réactionnel est ensuite refroidi à 40°±2°C puis 31.4 g de phosphate tripotassique et 0. 29 g d'iodure de sodium sont ajoutés.

Le milieu réactionnel est chauffé au reflux (108°/109°C) pendant 3 heures. Il est ensuite refroidi en 1 heure environ à 20°±2°C et maintenu à cette température pendant une nuit. 250 mL d'eau déminéralisée sont rajoutés à 20°±2°C et le tout est maintenu sous agitation à cette température pendant 30 minutes. La phase aqueuse est décantée et la phase organique est lavée avec 2 X 250mL d'eau déminéralisée.

La solution organique est concentrée à pression ordinaire à 100 ml.

On obtient une solution jaune à laquelle on ajoute 500 mL de toluène puis le milieu réactionnel est distillé à volume constant, à pression ordinaire en maintenant à niveau par rajout de toluène (350 mL). La température en fin d'entraînement est 110°C.

Puis le milieu réactionnel est refroidi à 20°±2°C, on ajoute en 5 minutes environ 10 ml d'une solution à 62 % dans l'eau d'acide bromhydrique.

La cristallisation est amorcée avec 0.01 g de bromhydrate de 1-[bis-(4-chlorophényl)méthyl]azétidin-3-ol.

Le milieu est maintenu sous agitation, sous azote à 20°±2°C pendant 30 minutes. Puis on ajoute en 5 minutes environ 10 ml d'une solution à 62 % dans l'eau d'acide bromhydrique sous agitation, sous azote à 20°±2°C. Ce mélange est maintenu à cette température pendant 30 min. Puis le milieu est essoré et lavé par déplacement avec 2 x 50 mL de toluène

On obtient ainsi le bromhydrate de 1-[bis-(4-chlorophényl)méthyl]azétidin-3-ol avec un rendement de 75.8 %.

### Etape 5

Dans un tétracol de 1 litre muni d'une agitation mécanique, d'une sonde thermométrique, et placé sous atmosphère d'azote, on introduit 50 g de bromhydrate de 1-[bis-(4-chlorophényl)méthyl]azétidin-3-ol, 250 ml de chlorure de méthylène à 20±2°C puis en 5 minutes, 53.6 mL de triéthylamine. La température évolue jusqu'à 27°C. On Agite pendant 30 minutes en laissant évoluer la température qui descend à +22°C. Le milieu est refroidi à -10°±2°C et 14.9mL de chlorure de méthane sulfonyle est ajouté à -10°±2°C en 15 à 30 minutes environ. La suspension blanche est maintenue pendant 2 heures à -10°±2°C. On ajoute 200 mL d'une solution à 50 g/l d'hydrogénocarbonate de sodium en 10 minutes en laissant monter la température à +10°/+15°C. La dissolution est totale et le milieu est maintenu à +10°/+15°C pendant 30 minutes. Le milieu est décanté et la solution organique est lavée avec 2 x 50mL d'une solution à 50 g/l d'hydrogénocarbonate de sodium à +10°/+15°C. La solution chlorométhylénique de méthylsulfonate de 1-[bis(4-chlorophényl)méthyl]azétidin-3-yle est concentrée sous agitation, sous vide de 50 mbar jusqu'à 100ml.

### Etape 6

A la solution chlorométhylénique de méthylsulfonate de 1-[bis(4-chlorophényl)méthyl]azétidin-3-yle on ajoute 425 mL de toluène.

On distille sous agitation, sous vide de 50 mbar à température intérieure inférieure à 60°C en maintenant à niveau constant par rajout de 200 mL de toluène. La solution est refroidie sous agitation, sous azote à 20°C et 32.7 g de phosphate tripotassique anhydre, 8 mL de tris(dioxa-3,6-heptyl)amine et 27 g de N-(3,5-difluorophényl)méthylsulfonamide sont ajoutés. Cette suspension est chauffée au reflux (112°C) pendant 20 heures. Le milieu est refroidi à 25°C. 250 mL d'eau déminéralisée sont ajoutés en une fois. Le milieu est décanté et la phase organique est lavée avec 2 x 250mL d'eau déminéralisée.

La phase organique est concentrée à 100 mL, sous vide de 50 mbars à température inférieure à 50°C. On ajoute 750 mL d'alcool isopropylique et on poursuit la distillation sous sous vide en maintenant à niveau constant par rajout de 500 mL d'alcool isopropylique. La recristallisation débute en cours de distillation. Le milieu est chauffé au reflux pour dissoudre en majorité le produit puis est refroidi sous agitation, sous azote à 20°C, en 30 minutes environ (recristallisation vers 70°C) et est maintenu pendant 1 heure dans ces conditions. Après essorage et lavage avec de l'alcool isopropylique (2 x 50mL) on obtient le N-{1-[bis-(4-chlorophényl)méthyl]azétidin-3-yl}-N-(3,5-difluorophényl)-méthylsulfonamide avec 79.2 % de rendement.

### Accès au composé IX

Dans un tétracol de 100 mL muni d'une agitation magnétique, d'une sonde thermométrique, d'une arrivée d'azote et d'une sortie avec barbotage, on introduit 10 g de Bis-(4-chloro-phenyl)-methanone, 30 g de Formamide et 0.81 g de chlorure de magnésium hexahydrate. La suspension est chauffée à 170°±5°C pendant environ 3 heures.

Le milieu est refroidi vers 100°C puis 30 ml d'eau sont introduits en 15 min environ sous bonne agitation. La masse cristallise au cours de l'addition d'eau et après refroidissement vers 20°±5°C, le milieu est essoré et lavé par déplacement avec 3 x 10 mL d'eau.

On obtient le N-[Bis-(4-chloro-phenyl)-methyl]-formamide avec un rendement de 93%

### Accès au composé V

Dans un tétracol de 1 litre muni d'une agitation mécanique type demi-lune, d'une sonde thermométrique, d'une arrivée d'azote et d'une sortie avec barbotage, on introduit 50 g de N-[Bis-(4-chloro-phenyl)-methyl]-formamide et 250 mL de méthanol puis 23 mL d'acide chlorhydrique 37 %. Le milieu est chauffé au reflux du méthanol pendant environ 2 heures. Après refroidissement à 65°±5°C, le milieu est neutralisé par addition en 30 min environ d'une solution de 38.2 g de carbonate de sodium dans 250 ml d'eau. Après refroidissement à 10°±2°C, le milieu est essoré et lavé par déplacement avec 2 x 20 mL d'eau.

On obtient le [Bis-(4-chlorophenyl)methyl]-amine à l'état de base avec un rendement de 98 %

## Revendications

1. Procédé de synthèse de dérivés de formule générale (I) dans laquelle R, R' et R" représentent indépendamment l'un de l'autre un ou plusieurs radicaux hydrogène, halogène (Cl, F, Br, I), cyano, nitro, alkyle linéaire ou ramifié de 1 à 6 atomes de carbone, alkoxy linéaire ou ramifié de 1 à 6 atomes de carbone, carboxylate d'alkyle linéaire ou ramifié de 1 à 6 atomes de carbone, trifluorométhyle, trifluorométhoxy et R"' représente un groupe alkyle ou perfluoroalkyle linéaire ou ramifié de 1 à 6 atomes de carbone, ou un groupement aryle éventuellement substitué par un ou plusieurs radicaux R" 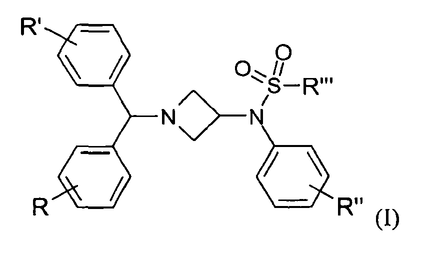 **caractérisé en ce que** l'on condense un sulfonate de formule (II) dans laquelle R, R' représentent indépendamment l'un de l'autre un ou plusieurs radicaux hydrogène, halogène (Cl, F, Br, I), cyano, nitro, alkyle linéaire ou ramifié de 1 à 6 atomes de carbone, alkoxy linéaire ou ramifié de 1 à 6 atomes de carbone, carboxylate d'alkyle linéaire ou ramifié de 1 à 6 atomes de carbone, trifluorométhyle, trifluorométhoxy et R1 représente un radical méthyle, trifluoromethyle, C₄F₉, C₈F₁₇ ou phényle éventuellement substitué par un reste méthyle, bromo ou nitro 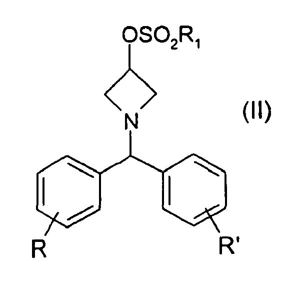 avec une sulfonamide de formule (III) dans laquelle R" représente un ou plusieurs radicaux hydrogène, halogène (Cl, F, Br, I), cyano, nitro, alkyle linéaire ou ramifié de 1 à 6 atomes de carbone, alkoxy linéaire ou ramifié de 1 à 6 atomes de carbone, carboxylate
d'alkyle linéaire ou ramifié de 1 à 6 atomes de carbone, trifluorométhyle, trifluorométhoxy et R"' représente un groupe alkyle ou perfluoroalkyle linéaire ou ramifié de 1 à 6 atome de carbone, ou un groupement aryle éventuellement substitué par un ou plusieurs radicaux R" 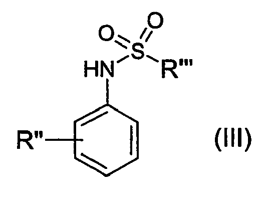 dans un solvant organique éventuellement en présence d'eau, à une température comprise entre 20°C et 150°C en présence d'une base minérale ou organique et d'un agent de transfert de phase.

2. Procédé de synthèse selon la revendication 1 **caractérisé en ce que** R et R' représentent des chlores en position para, R" représente un fluor en position 3 et 5 et R"' représente un méthyle pour former le N-{1-[bis-(4-chlorophényl)méthyl]azétidin-3-yl}-N-(3,5-difluorophényl)-méthylsulfonamide.

3. Procédé de synthèse selon la revendication 1 **caractérisé en ce que** R1 est un radical méthyle.

4. Procédé de synthèse selon la revendication 1 **caractérisé en ce que** le solvant est soit le toluène, le xylène, l'heptane, l'hexane, un solvant éthéré tel que le tetrahydrofuranne, le diméthoxyéthane, ou encore un solvant chloré tel que le mono ou le dichlorobenzène, le chlorobutane ou le chlorure de méthylène.

5. Procédé de synthèse selon la revendication 1 **caractérisé en ce que** la base minérale ou organique est soit un hydrogénocarbonate, un carbonate, un phosphate, un hydroxyde d'un métal alcalin tel que le lithium, le sodium, le potassium ou le césium et particulièrement de phosphate tripotassique anhydre, un alkoxyde de métaux alcalins tels que le méthylate, l'éthylate, le t-amylate ou le t-butylate de sodium ou de potassium ou des bases aminées telles que le DBU, DBN ou la tétraméthylguanidine.

6. Procédé de synthèse selon la revendication 1 **caractérisé en ce que** l'agent de transfert de phase est soit un éther couronne tel que le 12C4, 15C5, 18C6, le pentaglyme (Glyme-6) ou le polyéthylène glycol PEG 400 ou le tris(dioxa-3,6-heptyl)amine (TDA-1)

7. Procédé de synthèse selon la revendication 1 **caractérisé en ce que** R, R'représentent des chlores en position para et R" représente un fluor en position 3 et 5 et R"' est un méthyle, le solvant est le toluène, la base le phosphate tripotassique et l'agent de transfert de phase le tris(dioxa-3,6-heptyl)amine (TDA-1) pour former le N-{1-[bis-(4-chlorophényl)méthyl]azétidin-3-yl}-N-(3,5-difluorophényl)-méthylsulfonamide.

8. Procédé de synthèse selon la revendication 1 **caractérisé en ce qu'**un produit de formule (IV) 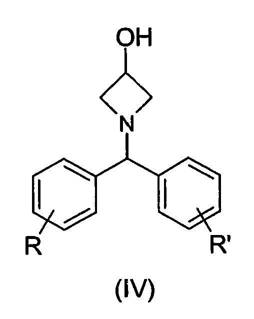 dans laquelle R et R' représentent indépendamment l'un de l'autre un ou plusieurs radicaux hydrogène, halogène (Cl, F, Br, I), cyano, nitro, alkyle linéaire ou ramifié de 1 à 6 atomes de carbone, alkoxy linéaire ou ramifié de 1 à 6 atomes de carbone, carboxylate d'alkyle linéaire ou ramifié de 1 à 6 atomes de carbone, trifluorométhyle, trifluorométhoxy est préparé en traitant un produit de formule (V) ou un de ses sels d'acide 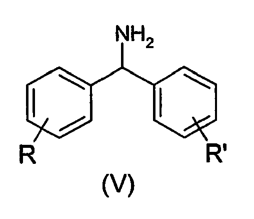 dans laquelle R et R' représentent indépendamment l'un de l'autre un ou plusieurs radicaux hydrogène, halogène (Cl, F, Br, I), cyano, nitro, alkyle linéaire ou ramifié de 1 à 6 atomes de carbone, alkoxy linéaire ou ramifié de 1 à 6 atomes de carbone, carboxylate d'alkyle linéaire ou ramifié de 1 à 6 atomes de carbone, trifluorométhyle, trifluorométhoxy, avec de l'épibromhydrine ou épichlorhydrine à une température comprise entre 20°C et 150 °C dans un solvant organique en présence de base, d'un additif et le cas échéant d'une phase aqueuse, le produit de formule (IV) obtenu permettant de préparer un produit de formule (II).

9. Procédé de synthèse selon la revendication 1, **caractérisé en ce que** la synthèse de la [Bis-(4-chlorophenyl)methyl]-amine comprend les étapes suivantes :
a) formation du N-[Bis-(4-chloro-phenyl)-methyl]-formamide à partir de la Bis-(4-chloro-phenyl)-methanone en présence de formamide avec une catalyse au chlorure de magnésium ;
b) déformylation en présence de HCl dans du méthanol pour donner la [Bis-(4-chlorophenyl)methyl]-amine ;
la [Bis-(4-chlorophenyl)methyl]-amine obtenue permettant de préparer un produit de formule (II).

10. Procédé de synthèse selon la revendication 8 **caractérisé en ce que** la base est le phosphate tripotassique anhydre.

11. Procédé de synthèse selon la revendication 8 **caractérisé en ce que** l'additif est l'iodure de sodium.

12. Procédé de synthèse selon la revendication 8 **caractérisé en ce que** le solvant est le n-butanol.

13. Procédé de synthèse selon la revendication 8 **caractérisé en ce qu'**un produit de formule (VI) 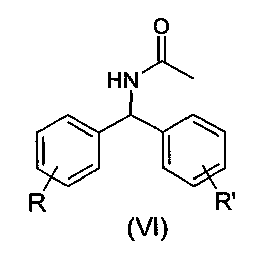 dans laquelle R et R' représentent indépendamment l'un de l'autre un ou plusieurs radicaux hydrogène, halogène (Cl, F, Br, I), cyano, nitro, alkyle linéaire ou ramifié de 1 à 6 atomes de carbone, alkoxy linéaire ou ramifié de 1 à 6 atomes de carbone, carboxylate d'alkyle linéaire ou ramifié de 1 à 6 atomes de carbone, trifluorométhyle, trifluorométhoxy est préparé en faisant réagir un produit de formule (VII) 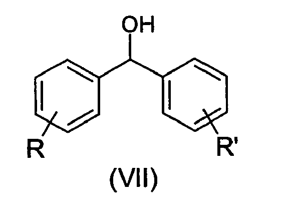 dans laquelle R et R' représentent indépendamment l'un de l'autre un ou plusieurs radicaux hydrogène, halogène (Cl, F, Br, I), cyano, nitro, alkyle linéaire ou ramifié de 1 à 6 atomes de carbone, alkoxy linéaire ou ramifié de 1 à 6 atomes de carbone, carboxylate d'alkyle linéaire ou ramifié de 1 à 6 atomes de carbone, trifluorométhyle, trifluorométhoxy avec un nitrile en présence d'une catalyse acide à une température comprise entre 50°C et 150°C, le produit de formule (VI) obtenu permettant de préparer un produit de formule (V).

14. Procédé de synthèse selon la revendication 13 **caractérisé en ce que** le nitrile est l'acétonitrile.

15. Procédé de synthèse selon la revendication 13 **caractérisé en ce que** l'acide est l'acide formique.

16. Procédé de synthèse selon la revendication 1 **caractérisé en ce qu'**un produit de formule (IV) 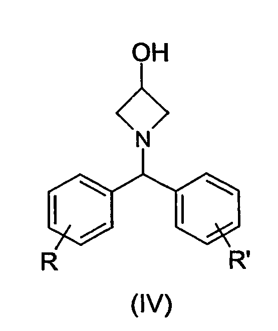 dans laquelle R et R' représentent indépendamment l'un de l'autre un ou plusieurs radicaux hydrogène, halogène (Cl, F, Br, I), cyano, nitro, alkyle linéaire ou ramifié de 1 à 6 atomes de carbone, alkoxy linéaire ou ramifié de 1 à 6 atomes de carbone, carboxylate d'alkyle linéaire ou ramifié de 1 à 6 atomes de carbone, trifluorométhyle, trifluorométhoxy, est préparé en trois étapes :
a) un produit de formule (VII) 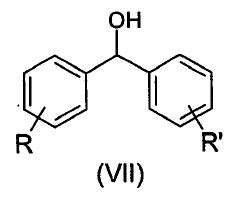 dans laquelle R et R' représentent indépendamment l'un de l'autre un ou plusieurs radicaux hydrogène, halogène (Cl, F, Br, I), cyano, nitro, alkyle linéaire ou ramifié de 1 à 6 atomes de carbone, alkoxy linéaire ou ramifié de 1 à 6 atomes de carbone, carboxylate d'alkyle linéaire ou ramifié de 1 à 6 atomes de carbone, trifluorométhyle, trifluorométhoxy réagit avec de l'acétonitrile en présence d'une catalyse par de l'acide formique à une température comprise entre 50°C et 150°C pour donner le produit de formule (VI) 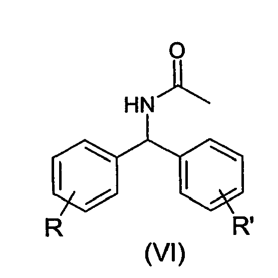 dans laquelle R et R' représentent indépendamment l'un de l'autre un ou plusieurs radicaux hydrogène, halogène (Cl, F, Br, I), cyano, nitro, alkyle linéaire ou ramifié de 1 à 6 atomes de carbone, alkoxy linéaire ou ramifié de 1 à 6 atomes de carbone, carboxylate d'alkyle linéaire ou ramifié de 1 à 6 atomes de carbone, trifluorométhyle, trifluorométhoxy ;
b) le produit de formule (VI) est transformé en chauffant et en milieu acide en un produit de formule (V) ou un de ses sels d'acide 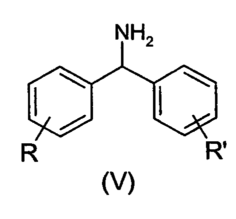 dans laquelle R et R' représentent indépendamment l'un de l'autre un ou plusieurs radicaux hydrogène, halogène (Cl, F, Br, I), cyano, nitro, alkyle linéaire ou ramifié de 1 à 6 atomes de carbone, alkoxy linéaire ou ramifié de 1 à 6 atomes de carbone, carboxylate d'alkyle linéaire ou ramifié de 1 à 6 atomes de carbone, trifluorométhyle, trifluorométhoxy,
c) le produit de formule (V) est transformé en produit de formule (IV) dans un solvant organique le n-butanol en présence de base le phosphate tripotassique anhydre, d'un additif l'iodure de sodium et le cas échéant d'une phase aqueuse, ledit produit de formule (IV) obtenu permettant de préparer un produit de formule (II).

## Claims

1. Process for synthesizing derivatives of general formula (I), in which R, R' and R" represent, independently of each other, one or more hydrogen, halogen (Cl, F, Br or I), cyano, nitro, linear or branched alkyl of 1 to 6 carbon atoms, linear or branched alkoxy of 1 to 6 carbon atoms, linear or branched alkyl carboxylate of 1 to 6 carbon atoms, trifluoromethyl or trifluoromethoxy radicals and R''' represents a linear or branched alkyl or perfluoroalkyl group of 1 to 6 carbon atoms or an aryl group optionally substituted with one or more radicals R" 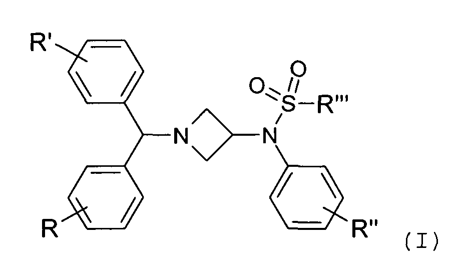 **characterized in that** a sulfonate of formula (II) in which R and R' represent, independently of each other, one or more hydrogen, halogen (Cl, F, Br or I), cyano, nitro, linear or branched alkyl of 1 to 6 carbon atoms, linear or branched alkoxy of 1 to 6 carbon atoms, linear or branched alkyl carboxylate of 1 to 6 carbon atoms, trifluoromethyl or trifluoromethoxy radicals and R1 represents a methyl, trifluoromethyl, C₄F₉, C₈F₁₇ or phenyl radical optionally substituted with a methyl, bromo or nitro residue 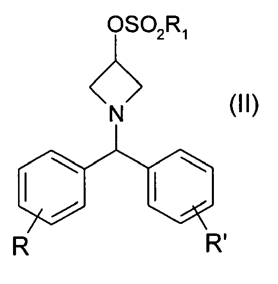 is condensated with a sulfonamide of formula (III) in which R" represents one or more hydrogen, halogen (Cl, F, Br or I), cyano, nitro, linear or branched alkyl of 1 to 6 carbon atoms, linear or branched alkoxy of 1 to 6 carbon atoms, linear or branched alkyl carboxylate of 1 to 6 carbon atoms, trifluoromethyl or trifluoromethoxy radicals and R''' represents a linear or branched alkyl or perfluoroalkyl group of 1 to 6 carbon atoms or an aryl group optionally substituted with one or more radicals R" 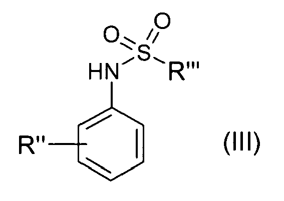 in an organic solvent optionally in the presence of water, at a temperature of between 20°C and 150°C in the presence of a mineral or organic base and of a phase-transfer agent.

2. Synthetic process according to Claim 1, **characterized in that** R and R' represent chlorines in the position para, R'' represents a fluorine in positions 3 and 5, and R''' represente a methyl, to form N-{1-[bis(4-chlorophenyl)methyl]azetidin-3-yl}-N-(3,5-difluorophenyl)methylsulfonamide.

3. Synthetic process according to Claim 1, **characterized in that** R1 is a methyl radical.

4. Synthetic process according to Claim 1, **characterized in that** the solvent is either toluene, xylene, heptane, hexane, an ether solvent such as tetrahydrofuran or dimethoxyethane, or alternatively a chlorinated solvent such as monochlorobenzene, dichlorobenzene, chlorobutane or methylene chloride.

5. Synthetic process according to Claim 1, **characterized in that** the mineral or organic base is either a hydrogen carbonate, carbonate, phosphate or hydroxide of an alkali metal such as lithium, sodium, potassium or caesium, and particularly anhydrous tripotassium phosphate, an alkali metal alkoxide such as sodium or potassium methoxide, ethoxide, t-amyloxide or t-butoxide, or amine bases such as DBU, DBN or tetramethylguanidine.

6. Synthetic process according to Claim 1, **characterized in that** the phase-transfer agent is a crown ether such as 12-C-4, 15-C-5 or 18-C-6, pentaglyme (Glyme-6) or polyethylene glycol PEG 400, or tris(dioxa-3,6-heptyl)amine (TDA-1).

7. Synthetic process according to Claim 1, **characterized in that** R and R' represent chlorines in the para position and R" represents a fluorine in positions 3 and 5, and R''' is a methyl, the solvent is toluene, the base is tripotassium phosphate and the phase-transfer agent is tris(dioxa-3,6-heptyl)amine (TDA-1), to form N-{1-[bis(4-chlorophenyl)methyl]azetidin-3-yl}-N-(3,5-difluorophenyl)methylsulfonamide.

8. Synthetic process according to Claim 1, **characterized in that** a product of formula (IV) 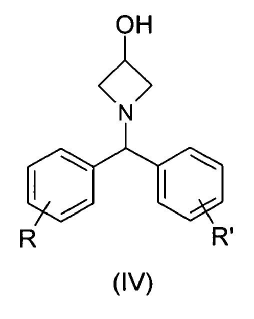 in which R and R' represent, independently of each other, one or more hydrogen, halogen (Cl, F, Br or I), cyano, nitro, linear or branched alkyl of 1 to 6 carbon atoms, linear or branched alkoxy of 1 to 6 carbon atoms, linear or branched alkyl carboxylate of 1 to 6 carbon atoms, trifluoromethyl or trifluoromethoxy radicals, is prepared by treating a product of formula (V), or an acid salt thereof 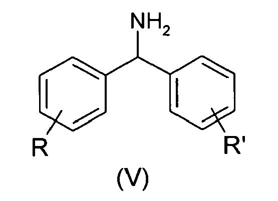 in which R and R' represent, independently of each other, one or more hydrogen, halogen (Cl, F, Br or I), cyano, nitro, linear or branched alkyl of 1 to 6 carbon atoms, linear or branched alkoxy of 1 to 6 carbon atoms, linear or branched alkyl carboxylate of 1 to 6 carbon atoms, trifluoromethyl or trifluoromethoxy radicals, with epibromohydrin or epichlorohydrin at a temperature of between 20°C and 150°C in an organic solvent in the presence of base, an additive and, where appropriate, an aqueous phase, the product of formula (IV) obtained making it possible to prepare a product of formula (II).

9. Synthetic process according to Claim 1, **characterized in that** the synthesis of [bis(4-chlorophenyl)methyl]amine comprises the following steps:
a) formation of N-[bis-(4-chlorophenyl)-methyl]formamide from bis(4-chloro-phenyl)methanone in the presence of formamide, with catalysis using magnesium chloride,
b) deformylation in the presence of HCl in methanol, to give [bis(4-chlorophenyl)methyl]amine,
the [bis(4-chlorophenyl)methyl]amine obtained making it possible to prepare a product of formula (II).

10. Synthetic process according to Claim 8, **characterized in that** the base is anhydrous tripotassium phosphate.

11. Synthetic process according to Claim 8, **characterized in that** the additive is sodium iodide.

12. Synthetic process according to Claim 8, **characterized in that** the solvent is n-butanol.

13. Synthetic process according to Claim 8, **characterized in that** a product of formula (VI) 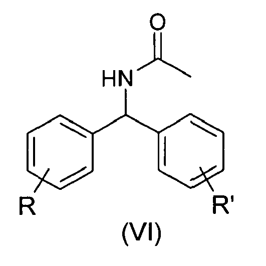 in which R and R' represent, independently of each other, one or more hydrogen, halogen (Cl, F, Br or I), cyano, nitro, linear or branched alkyl of 1 to 6 carbon atoms, linear or branched alkoxy of 1 to 6 carbon atoms, linear or branched alkyl carboxylate of 1 to 6 carbon atoms, trifluoromethyl or trifluoromethoxy radicals, is prepared by reacting a product of formula (VII) 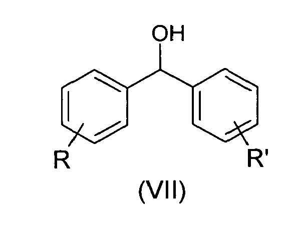 in which R and R' represent, independently of each other, one or more hydrogen, halogen (Cl, F, Br or I), cyano, nitro, linear or branched alkyl of 1 to 6 carbon atoms, linear or branched alkoxy of 1 to 6 carbon atoms, linear or branched alkyl carboxylate of 1 to 6 carbon atoms, trifluoromethyl or trifluoromethoxy radicals, with a nitrile in the presence of acid catalysis at a temperature of between 50°C and 150°C, the product of formula (VI) obtained making it possible to obtain a product of formula (V).

14. Synthetic process according to Claim 13, **characterized in that** the nitrile is acetonitrile.

15. Synthetic process according to Claim 13, **characterized in that** the acid is formic acid.

16. Synthetic process according to Claim 1, **characterized in that** a product of formula (IV) 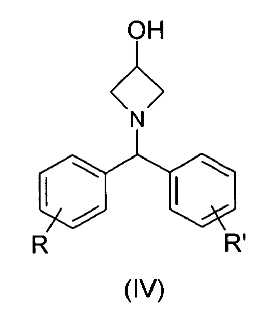 in which R and R' represent, independently of each other, one or more hydrogen, halogen (Cl, F, Br or I), cyano, nitro, linear or branched alkyl of 1 to 6 carbon atoms, linear or branched alkoxy of 1 to 6 carbon atoms, linear or branched alkyl carboxylate of 1 to 6 carbon atoms, trifluoromethyl or trifluoromethoxy radicals, is prepared in three steps:
a) a product of formula (VII) 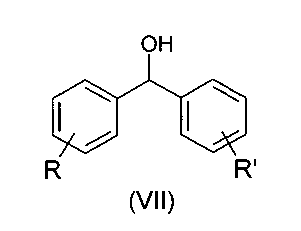 in which R and R' represent, independently of each other, one or more hydrogen, halogen (Cl, F, Br or I), cyano, nitro, linear or branched alkyl of 1 to 6 carbon atoms, linear or branched alkoxy of 1 to 6 carbon atoms, linear or branched alkyl carboxylate of 1 to 6 carbon atoms, trifluoromethyl or trifluoromethoxy radicals, is reacted with acetonitrile in the presence of catalysis with formic acid at a temperature of between 50°C and 150°C, to give the product of formula (VI) 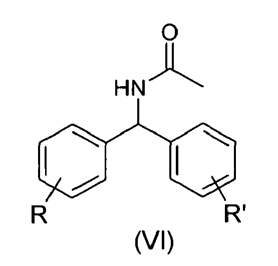 in which R and R' represent, independently of each other, one or more hydrogen, halogen (Cl, F, Br or I), cyano, nitro, linear or branched alkyl of 1 to 6 carbon atoms, linear or branched alkoxy of 1 to 6 carbon atoms, linear or branched alkyl carboxylate of 1 to 6 carbon atoms, trifluoromethyl or trifluoromethoxy radicals;
b) the product of formula (VI) is converted by heating in acidic medium into a product of formula (V), or an acid salt thereof 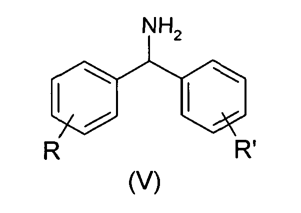 in which R and R' represent, independently of each other, one or more hydrogen, halogen (Cl, F, Br or I), cyano, nitro, linear or branched alkyl of 1 to 6 carbon atoms, linear or branched alkoxy of 1 to 6 carbon atoms, linear or branched alkyl carboxylate of 1 to 6 carbon atoms, trifluoromethyl or trifluoromethoxy radicals,
c) the product of formula (V) is converted into a product of formula (IV) in an organic solvent, n-butanol, in the presence of a base, anhydrous tripotassium phosphate, an additive, sodium iodide, and, where appropriate, an aqueous phase, said product of formula (IV) obtained making it possible to prepare a product of formula (II).

## Patentansprüche

1. Verfahren zur Synthese von Derivaten der allgemeinen Formel (I), worin R, R' und R'' unabhängig voneinander für einen oder mehrere Wasserstoffreste, Halogenreste (Cl, F, Br, I), Cyanoreste, Nitroreste, lineare oder verzweigte Alkylreste mit 1 bis 6 Kohlenstoffatomen, lineare oder verzweigte Alkoxyreste mit 1 bis 6 Kohlenstoffatomen, lineare oder verzweigte Alkylcarboxylatreste mit 1 bis 6 Kohlenstoffatomen, Trifluormethylreste oder Trifluormethoxyreste stehen und R''' für eine lineare oder verzweigte Alkyl- oder Perfluoralkylgruppe mit 1 bis 6 Kohlenstoffatomen oder eine gegebenenfalls durch einen oder mehrere Reste R'' substituierte Arylgruppe steht, 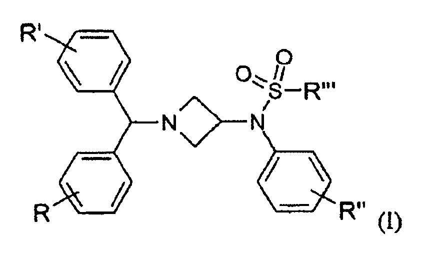 **dadurch gekennzeichnet, daß** man ein Sulfonat der Formel (II), worin R und R' unabhängig voneinander für einen oder mehrere Wasserstoffreste, Halogenreste (Cl, F, Br, I), Cyanoreste, Nitroreste, lineare oder verzweigte Alkylreste mit 1 bis 6 Kohlenstoffatomen, lineare oder verzweigte Alkoxyreste mit 1 bis 6 Kohlenstoffatomen, lineare oder verzweigte Alkylcarboxylatreste mit 1 bis 6 Kohlenstoffatomen, Trifluormethylreste oder Trifluormethoxyreste stehen und R1 für einen Methyl-, Trifluormethyl-, C₄F₉-, C₈F₁₇- oder gegebenenfalls durch einen Methyl-, Brom- oder Nitrorest substituierten Phenylrest steht, 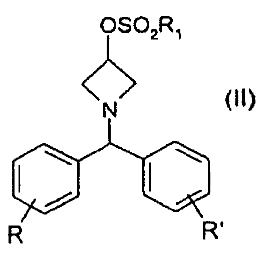 in einem organischen Lösungsmittel gegebenenfalls in Gegenwart von Wasser bei einer Temperatur zwischen 20°C und 150°C in Gegenwart einer anorganischen oder organischen Base und eines Phasentransferagens mit einem Sulfonamid der Formel (III), worin R'' unabhängig voneinander für einen oder mehrere Wasserstoffreste, Halogenreste (Cl, F, Br, I), Cyanoreste, Nitroreste, lineare oder verzweigte Alkylreste mit 1 bis 6 Kohlenstoffatomen, lineare oder verzweigte Alkoxyreste mit 1 bis 6 Kohlenstoffatomen, lineare oder verzweigte Alkylcarboxylatreste mit 1 bis 6 Kohlenstoffatomen, Trifluormethylreste oder Trifluormethoxyreste steht und R''' für eine lineare oder verzweigte Alkyl- oder Perfluoralkylgruppe mit 1 bis 6 Kohlenstoffatomen oder eine gegebenenfalls durch einen oder mehrere Reste R'' substituierte Arylgruppe steht, 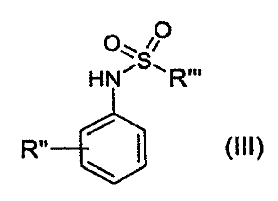 umsetzt.

2. Syntheseverfahren nach Anspruch 1, **dadurch gekennzeichnet, daß** R und R' für Chloratome in para-Position stehen, R'' für ein Fluor in Position 3 und 5 steht und R''' für Methyl steht, zur Bildung von N-{1-[Bis(4-chlorphenyl)methyl]-azetidin-3-yl}-N-(3,5-difluorphenyl)methylsulfonamid.

3. Syntheseverfahren nach Anspruch 1, **dadurch gekennzeichnet, daß** R1 für einen Methylrest steht.

4. Syntheseverfahren nach Anspruch 1, **dadurch gekennzeichnet, daß** es sich bei dem Lösungsmittel um Toluol, Xylol, Heptan, Hexan, ein Ether-Lösungsmittel wie Tetrahydrofuran oder Dimethoxyethan oder auch ein chloriertes Lösungsmittel wie Mono- oder Dichlorbenzol, Chlorbutan oder Methylenchlorid handelt.

5. Syntheseverfahren nach Anspruch 1, **dadurch gekennzeichnet, daß** es sich bei der anorganischen oder organischen Base um ein Hydrogencarbonat, ein Carbonat, ein Phosphat oder ein Hydroxid eines Alkalimetalls wie Lithium, Natrium, Kalium oder Caesium und insbesondere wasserfreies Trikaliumphosphat, ein Alkalimetallalkoxid wie Natrium-oder Kaliummethylat, -ethylat, -t-amylat oder -t-butylat oder Aminbasen wie DBU, DBN oder Tetramethylguanidin handelt.

6. Syntheseverfahren nach Anspruch 1, **dadurch gekennzeichnet, daß** es sich bei dem Phasentransferagens um einen Kronenether wie 12-C-4, 15-C-5, 18-C-6, Pentaglyme (Glyme-6) oder Polyethylenglykol PEG 400 oder Tris(dioxa-3,6-heptyl)amin (TDA-1) handelt.

7. Syntheseverfahren nach Anspruch 1, **dadurch gekennzeichnet, daß** R und R' für Chloratome in para-Position stehen und R'' für ein Fluor in Position 3 und 5 steht und R''' für Methyl steht, es sich bei dem Lösungsmittel um Toluol handelt, es sich bei der Base um Trikaliumphosphat handelt und es sich bei dem Phasentransferagens um Tris(dioxa-3,6-heptyl)amin (TDA-1) handelt, zur Bildung von N-{1-[Bis(4-chlorphenyl)methyl]-azetidin-3-yl}-N-(3,5-difluorphenyl)methylsulfonamid.

8. Syntheseverfahren nach Anspruch 1, **dadurch gekennzeichnet, daß** man ein Produkt der Formel (IV) 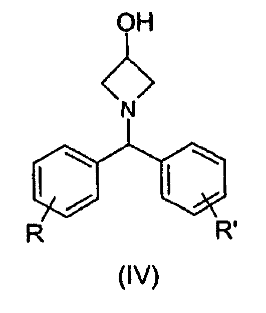 worin R und R' unabhängig voneinander für einen oder mehrere Wasserstoffreste, Halogenreste (C1, F, Br, I), Cyanoreste, Nitroreste, lineare oder verzweigte Alkylreste mit 1 bis 6 Kohlenstoffatomen, lineare oder verzweigte Alkoxyreste mit 1 bis 6 Kohlenstoffatomen, lineare oder verzweigte Alkylcarboxylatreste mit 1 bis 6 Kohlenstoffatomen, Trifluormethylreste oder Trifluormethoxyreste stehen, herstellt, indem man ein Produkt der Formel (V) oder ein Säuresalz davon 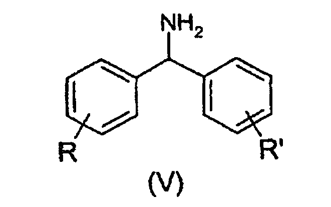 worin R und R' unabhängig voneinander für einen oder mehrere Wasserstoffreste, Halogenreste (Cl, F, Br, I), Cyanoreste, Nitroreste, lineare oder verzweigte Alkylreste mit 1 bis 6 Kohlenstoffatomen, lineare oder verzweigte Alkoxyreste mit 1 bis 6 Kohlenstoffatomen, lineare oder verzweigte Alkylcarboxylatreste mit 1 bis 6 Kohlenstoffatomen, Trifluormethylreste oder Trifluormethoxyreste stehen, bei einer Temperatur zwischen 20°C und 150°C in einem organischen Lösungsmittel in Gegenwart von Base, einem Additiv und gegebenenfalls einer wäßrigen Phase mit Epibromhydrin oder Epichlorhydrin behandelt, wobei das erhaltene Produkt der Formel (IV) die Herstellung eines Produkts der Formel (II) ermöglicht.

9. Syntheseverfahren nach Anspruch 1, **dadurch gekennzeichnet, daß** die Synthese von [Bis(4-chlorphenyl)methyl]amin die folgenden Schritte umfaßt:
a) Bildung von N-[Bis-(4-chlorphenyl)methyl]-formamid aus Bis(4-chlorphenyl)methanon in Gegenwart von Formamid unter Magnesiumchlorid-Katalyse;
b) Deformylierung in Gegenwart von HCl in Methanol zu [Bis(4-chlorphenyl)methyl]amin;
wobei das erhaltene [Bis(4-chlorphenyl)methyl]amin die Herstellung eines Produkts der Formel (II) ermöglicht.

10. Syntheseverfahren nach Anspruch 8, **dadurch gekennzeichnet, daß** es sich bei der Base um wasserfreies Trikaliumphosphat handelt.

11. Syntheseverfahren nach Anspruch 8, **dadurch gekennzeichnet, daß** es sich bei dem Additiv um Natriumiodid handelt.

12. Syntheseverfahren nach Anspruch 8, **dadurch gekennzeichnet, daß** es sich bei dem Lösungsmittel um n-Butanol handelt.

13. Syntheseverfahren nach Anspruch 8, **dadurch gekennzeichnet, daß** man ein Produkt der Formel (VI) 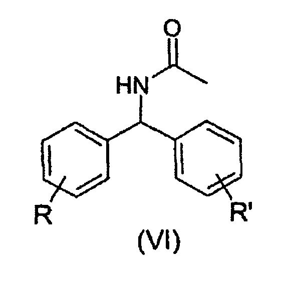 worin R und R' unabhängig voneinander für einen oder mehrere Wasserstoffreste, Halogenreste (C1, F, Br, I), Cyanoreste, Nitroreste, lineare oder verzweigte Alkylreste mit 1 bis 6 Kohlenstoffatomen, lineare oder verzweigte Alkoxyreste mit 1 bis 6 Kohlenstoffatomen, lineare oder verzweigte Alkylcarboxylatreste mit 1 bis 6 Kohlenstoffatomen, Trifluormethylreste oder Trifluormethoxyreste stehen, herstellt, indem man ein Produkt der Formel (VII) 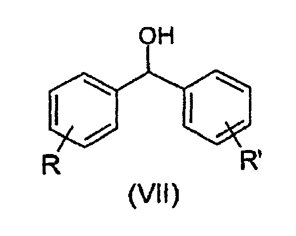 worin R und R' unabhängig voneinander für einen oder mehrere Wasserstoffreste, Halogenreste (Cl, F, Br, I), Cyanoreste, Nitroreste, lineare oder verzweigte Alkylreste mit 1 bis 6 Kohlenstoffatomen, lineare oder verzweigte Alkoxyreste mit 1 bis 6 Kohlenstoffatomen, lineare oder verzweigte Alkylcarboxylatreste mit 1 bis 6 Kohlenstoffatomen, Trifluormethylreste oder Trifluormethoxyreste stehen, in Gegenwart von Säurekatalyse bei einer Temperatur zwischen 50°C und 150°C mit einem Nitril umsetzt, wobei das erhaltene Produkt der Formel (VI) die Herstellung einer Verbindung der Formel (V) ermöglicht.

14. Syntheseverfahren nach Anspruch 13, **dadurch gekennzeichnet, daß** es sich bei dem Nitril um Acetonitril handelt.

15. Syntheseverfahren nach Anspruch 13, **dadurch gekennzeichnet, daß** es sich bei der Säure um Ameisensäure handelt.

16. Syntheseverfahren nach Anspruch 1, **dadurch gekennzeichnet, daß** man ein Produkt der Formel (IV) 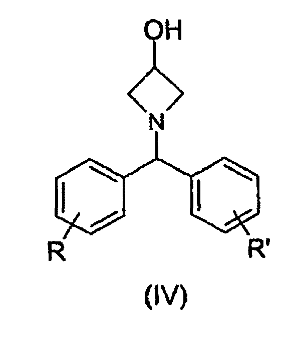 worin R und R' unabhängig voneinander für einen oder mehrere Wasserstoffreste, Halogenreste (Cl, F, Br, I), Cyanoreste, Nitroreste, lineare oder verzweigte Alkylreste mit 1 bis 6 Kohlenstoffatomen, lineare oder verzweigte Alkoxyreste mit 1 bis 6 Kohlenstoffatomen, lineare oder verzweigte Alkylcarboxylatreste mit 1 bis 6 Kohlenstoffatomen, Trifluormethylreste oder Trifluormethoxyreste stehen, in drei Schritten herstellt:
a) ein Produkt der Formel (VII) 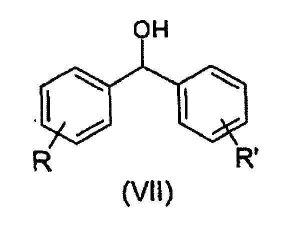 worin R und R' unabhängig voneinander für einen oder mehrere Wasserstoffreste, Halogenreste (Cl, F, Br, I), Cyanoreste, Nitroreste, lineare oder verzweigte Alkylreste mit 1 bis 6 Kohlenstoffatomen, lineare oder verzweigte Alkoxyreste mit 1 bis 6 Kohlenstoffatomen, lineare oder verzweigte Alkylcarboxylatreste mit 1 bis 6 Kohlenstoffatomen, Trifluormethylreste oder Trifluormethoxyreste stehen, wird in Gegenwart von Ameisensäure-Katalyse bei einer Temperatur zwischen 50°C und 150°C mit Acetonitril zu dem Produkt der Formel (VI) 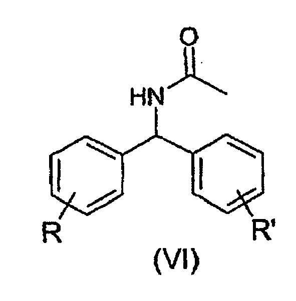 worin R und R' unabhängig voneinander für einen oder mehrere Wasserstoffreste, Halogenreste (Cl, F, Br, I), Cyanoreste, Nitroreste, lineare oder verzweigte Alkylreste mit 1 bis 6 Kohlenstoffatomen, lineare oder verzweigte Alkoxyreste mit 1 bis 6 Kohlenstoffatomen, lineare oder verzweigte Alkylcarboxylatreste mit 1 bis 6 Kohlenstoffatomen, Trifluormethylreste oder Trifluormethoxyreste stehen, umgesetzt;
b) das Produkt der Formel (VI) wird durch Erhitzen in saurem Medium in ein Produkt der Formel (V) oder ein Säuresalz davon 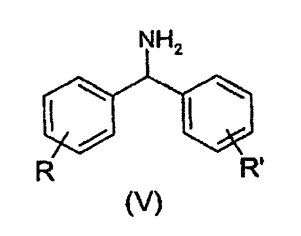 worin R und R' unabhängig voneinander für einen oder mehrere Wasserstoffreste, Halogenreste (Cl, F, Br, I), Cyanoreste, Nitroreste, lineare oder verzweigte Alkylreste mit 1 bis 6 Kohlenstoffatomen, lineare oder verzweigte Alkoxyreste mit 1 bis 6 Kohlenstoffatomen, lineare oder verzweigte Alkylcarboxylatreste mit 1 bis 6 Kohlenstoffatomen, Trifluormethylreste oder Trifluormethoxyreste stehen, umgewandelt;
c) das Produkt der Formel (V) wird in einem organischen Lösungsmittel, n-Butanol, in Gegenwart von wasserfreiem Trikaliumphosphat, einem Additiv, Natriumiodid, und gegebenenfalls einer wäßrigen Phase in ein Produkt der Formel (IV) umgewandelt, wobei das Produkt der Formel (IV) die Herstellung eines Produkts der Formel (II) ermöglicht.
